# EUROPÄISCHE PATENTSCHRIFT

(11) **EP 1 572 857 B1**
(45) Veröffentlichungstag und Bekanntmachung des Hinweises auf die Patenterteilung: **07.05.2008**
(21) Anmeldenummer: 03785760.4
(22) Anmeldetag: 06.12.2003
(51) Int. Cl.: C11D 17/00, A61K 8/03, A61Q 19/00, B01F 17/00

(54) **ZWEISCHICHTIGE TEMPORÄR EMULGIERBARE ZUSAMMENSETZUNGEN**
TEMPORARILY EMULSIFYING DOUBLE-LAYER COMPOUNDS
COMPOSITIONS BICOUCHES TEMPORAIREMENT EMULSIFIABLES

(30) Priorität: 17.12.2002 DE 10258827
(43) Veröffentlichungstag der Anmeldung: 14.09.2005
(73) Patentinhaber: Henkel Kommanditgesellschaft auf Aktien, 40589 Düsseldorf (DE)
(72) Erfinder: HLOUCHA, Matthias, 50677 Köln (DE); STRIEPLING, Gerd-Lothar, 40591 Düsseldorf (DE); HEIDE, Barbara, 47809 Krefeld (DE); FRISOLI, Christian, 41564 Kaarst (DE); SCHOLZ, Wolfhard, 47829 Krefeld (DE); CORTEKAR, Hans-Wolfgang, 42855 Remscheid (DE)
(86) Internationale Anmeldenummer: PCT/EP2003/013849
(87) Internationale Veröffentlichungsnummer: WO 2004/055151

(56) Entgegenhaltungen:
- EP-A- 1 108 421
- EP-A- 1 169 998
- EP-A- 1 327 439
- WO-A-02/15849
- WO-A-03/077879
- US-A1- 2002 098 209
- US-B1- 6 180 587
- DATABASE CHEMICAL ABSTRACTS [Online] retrieved from STN Database accession no. 103: 109 775 XP002274272 & JP 60 078907 A (KOBAYASHI KOSE CO., LTD) 4. Mai 1985 (1985-05-04)

## Beschreibung

Die vorliegende Erfindung betrifft wäßrige, mindestens ein Öl und mindestens einen Emulgator enthaltende, flüssige, visuell mindestens zweischichtige Zusammensetzungen, die mindestens eine kontinuierliche wäßrige Phase I und mindestens eine diskontinuierliche ölige Phase II aufweisen, wobei eine ölige Phase II zusammen mit einem Teil einer wäßrigen Phase I eine obere Emulsionsschicht, und ein weiterer Teil der wäßrigen Phase I eine untere wäßrige Schicht der Zusammensetzungen bildet, die Zusammensetzungen sich durch Schütteln temporär in visuell homogene Emulsionen überführen lassen und weiterhin mindestens ein wasserlösliches Polymer enthalten.

Der Verbraucher erwartet bei einem Reinigungs- oder Pflegemittel, insbesondere wenn es sich um ein kosmetisches Mittel handelt, in der Regel nicht nur eine funktionale Wirkung, sondern in gleicher Weise auch ein attraktives Aussehen und eine die Wirkung unterstreichende galenische Zubereitung des Mittels.

In den letzten Jahren fanden mehrphasige Zubereitungen ein zunehmendes Interesse, welche im Ruhezustand in Form mehrerer getrennter Phasen bzw. Schichten vorliegen. Diese Phasentrennung bietet einerseits den technischen Vorteil einer größeren Formulierungsfreiheit hinsichtlich ansonsten miteinander unzureichend kompatibler Rezepturbestandteile, und andererseits die Möglichkeit, der Formulierung bestimmte optische bzw. ästhetische Effekte zu verleihen. So können die unterschiedlichen Phasen beispielsweise unterschiedlich gefärbt werden, oder klare und milchige Phasen miteinander kombiniert werden. Ein derartiges Erscheinungsbild ist beispielsweise besonders gut geeignet, dem Verbraucher unterschiedliche Eigenschaften visuell zu vermitteln, was vor allem bei den sogenannten "two-in-one-Produkten" eine Rolle spielt.

Andererseits muß es dem Verbraucher möglich sein, eine solche Zubereitung ohne besonderen Aufwand aus der Verpackung, insbesondere einer Flasche, so entnehmen zu können, daß die Zusammensetzung der entnommenen Probe identisch ist mit der Zusammensetzung der gesamten Zubereitung. Dies kann am einfachsten gewährleistet werden, indem die mehrphasige Zubereitung durch kurzes Schütteln in eine makroskopisch einheitliche Form, z.B. eine einheitlich aussehende Emulsion, überführbar ist. Diese soll sich aus den vorher erläuterten Gründen im Ruhezustand so rasch wieder in die ursprüngliche mehrphasige Form zurückverwandeln, daß der Verbraucher auch bei häufigerer Anwendung eines solchen Produktes dieses jeweils wieder im entmischten Zustand vorfindet.

Das Dokument JP-A 60078907 beschreibt zweischichtige kosmetische Zubereitungen mit guter Lagerstabilität, die als wesentliche Komponenten ein bei gewöhnlicher Temperatur flüssiges Öl und ein nichtionisches Tensid enthalten, und die aus einer oberen emulgierten Schicht und einer unteren wäßrigen Schicht bestehen.

In dem Dokument JP-A 62263297 ist eine Waschmittelzubereitung beschrieben, die ein Tensid und ein wasserlösliches Polymer enthält. Beim Schütteln der Zusammensetzung bildet sich eine einheitlich aussehende Flüssigkeit, die sich beim Stehen in zwei flüssige Phasen auftrennt.

Die deutsche Patentanmeldung DE 19915837.1 beschreibt wäßrige Zubereitungen, die im Ruhezustand in Form von zwei oder mehr entmischten, wäßrigen, unter Bewegung zeitweise ineinander dispergierbaren Phasen vorliegen. Die Zubereitungen enthalten wenigstens eine gelöste, oberflächenaktive Verbindung und wenigstens eine gelöste Polymerverbindung, sowie zur Beschleunigung der Phasentrennung im Ruhezustand wenigstens einen wasserlöslichen ein- oder mehrwertigen Alkohol.

WO 02/15849 A2 offenbart zweischichtige Zusammensetzungen mit einer oberen wässrigen Schicht und einer unteren wässrigen Schicht, enthaltend 5 - 35 Gew.-% einer oberflächenaktiven Substanz, 1 - 12 Gew.-% eines Verdickers, 4 - 5 Gew.-% eines Polyalkylenglycols und weiterhin ein nicht-chelatierendes Mineralsalz, das in ausreichenden Mengen enthalten sein muss, um eine schnelle Phasentrennung zwischen oberer und unterer Schicht zu induzieren. Die Zusammensetzungen können Öle enthalten, die sich im Ruhezustand in der oberen (wässrigen) Phase befinden.

EP 1108421 A2 offenbart eine zweischichtige Zusammensetzung, bei der im Ruhezustand eine der Schichten eine kinetisch stabile Emulsion sein kann, die Öle und hochmolekulare Polyethylenglycole enthalten kann.

US 6180587 B1 und US 2002/0098209 A1 offenbaren zweischichtige Zusammensetzungen mit einem wasserlöslichen Polymer, wobei im Ruhezustand das Öl nicht in emulgierter Form, sondern als einzelne zusammenhängende Bulkphase vorliegt.

Die aus dem Stand der Technik bekannten Zusammensetzungen ermöglichen zwar die Formulierung von zweischichtigen Systemen aus einer wäßrigen Schicht und einer Emulsionsschicht, die beim Schütteln eine einheitlich aussehende Flüssigkeit bilden. Läßt man diese jedoch nach dem Schütteln ruhen, so erfolgt eine sehr langsame Phasentrennung, die typischerweise einen Zeitbedarf in der Größenordnung eines Tages benötigt. Selbst dann ist jedoch in vielen Fällen die wäßrige Schicht immer noch trüb, und die Grenze zwischen den beiden Schichten nicht scharf, was optisch unbefriedigend ist. Zudem kommt es in vielen Fällen beim Stehen dieser Zusammensetzungen zu einer teilweisen Koaleszenz der emulgierten Tröpfchen, was eine unerwünschte Bildung von "Fettaugen" oder sogar die Abscheidung einer Ölschicht auf der Oberfläche der Zusammensetzungen zur Folge hat.

Untersuchungen der Erfinder der vorliegenden Anmeldung zeigten, daß es bei der Formulierung derartiger Zusammensetzungen einen Zielkonflikt gibt zwischen der Stabilität der Emulsionsphase gegenüber einer Koagulation und der Geschwindigkeit der Phasentrennung nach dem Schütteln. Dies bedeutet, daß Zusammensetzungen mit einer hohen Stabilität der Emulsionsphase den Nachteil einer sehr langsamen Phasentrennung nach dem Schütteln aufweisen und umgekehrt.

Der vorliegenden Erfindung lag somit das Problem zugrunde, eine lagerstabile, im Ruhezustand visuell zweischichtig erscheinende Zusammensetzung zur Verfügung zu stellen, wobei die eine Schicht klar sein sollte, die andere Schicht jedoch eine milchig erscheinende Emulsion darstellen sollte. Diese Zusammensetzung sollte sich durch Schütteln in eine einheitlich aussehende Dispersion überführen lassen, welche sich beim Stehen relativ rasch, insbesondere innerhalb eines Zeitraums von der Größenordnung einer Stunde, wieder in die ursprünglich vorhandenen Schichten auftrennt. Dabei war es zusätzlich wünschenswert, daß die ursprüngliche Klarheit der wäßrigen Schicht wiederhergestellt wurde, und daß die klare und die Emulsionsschicht wieder optisch deutlich voneinander abgegrenzt waren. Die Zusammensetzung sollte weiterhin eine gute Lagerstabilität aufweisen, worunter insbesondere eine hohe Stabilität der Emulsion gegenüber einer Koaleszenz der emulgierten Tröpfchen zu verstehen ist.

Die Erfinder der vorliegenden Anmeldung fanden nach umfangreichen Untersuchungen heraus, daß diese Aufgabenstellung gelöst wird durch die Gegenstände der Patentansprüche.

Gegenstand der Erfindung ist daher in einer ersten Ausführungsform eine wäßrige, flüssige, visuell mindestens zweischichtige Zusammensetzung, die mindestens eine kontinuierliche wäßrige Phase I und mindestens eine diskontinuierliche ölige Phase II aufweist, wobei eine ölige Phase II zusammen mit einem Teil einer wäßrigen Phase I eine obere Emulsionsschicht, und ein weiterer Teil der wäßrigen Phase I eine untere wäßrige Schicht der Zusammensetzung bildet, die Zusammensetzung sich durch Schütteln temporär in eine optisch einheitlich aussehende Emulsion überführen lässt, wobei die Zusammensetzung enthält:
5 - 40 Gew.-% mindestens eines Öls,
0,02 - 5 Gew.-% mindestens eines Emulgators,
weiterhin 0,5 - 20 Gew.-% mindestens eines wasserlöslichen Polymers und bis zu 5 Gew.-% eines Salzes oder eines Gemisches mehrerer Salze aus der Gruppe der Alkalimetall-, Erdalkalimetall- und Ammoniumsalze der Schwefelsäure, der Halogenwasserstoffsäuren sowie der Genußsäuren, sowie deren Mischungen, wobei sich die Prozentangaben jeweils auf das Gesamtgewicht der Zusammensetzung beziehen.

Der Begriff Schütteln ist in diesem Zusammenhang als Einbringung mechanischer Energie zu verstehen, was beispielsweise durch Schütteln von Hand erfolgen kann.

Im einfachsten Fall ist eine erfindungsgemäße Zusammensetzung zweischichtig und besteht aus einer kontinuierlichen wäßrigen Phase I und einer diskontinuierlichen öligen Phase II, wobei die ölige Phase II zusammen mit einem Teil der wäßrigen Phase I die obere Emulsionsschicht, und der verbleibende Teil der wäßrigen Phase I die untere wäßrige Schicht der Zusammensetzung bildet.

Aus dem Stand der Technik sind zahlreiche zweiphasige Zusammensetzungen bekannt, in welchen die beiden Phasen nicht nur visuell, sondern durch eine physikalische Grenzschicht voneinander getrennt sind, wie sie beispielsweise zwischen einer Öl- und einer Wasserphase vorliegt.

In den erfindungsgemäßen Zusammensetzungen erstreckt sich die kontinuierliche wäßrige Phase dagegen über das gesamte Volumen der Zusammensetzung, und die diskontinuierliche Ölphase liegt nur in einem Teil, und zwar im oberen Teil (obere Schicht) der Zusammensetzung vor. Die Grenzschicht, welche hier diese obere Schicht vom Rest der Zusammensetzung trennt, ist demgemäß lediglich eine visuell erscheinende Grenzschicht, da sich die wäßrige untere Schicht auch in die obere Schicht hinein erstreckt. Dieser Sachverhalt wurde durch Leitfähigkeitsmessungen an unterschiedlichen Stellen der erfindungsgemäßen Zusammensetzungen herausgefunden.

Unter einer visuell zweischichtigen Zusammensetzung ist im Sinne der Erfindung zu verstehen, daß zwei mit dem Auge optisch unterscheidbare Schichten vorliegen, die durch eine horizontal verlaufende Grenze voneinander getrennt sind. Diese Grenze ist als eine visuell wahrnehmbare Grenze zu verstehen, die nicht notwendigerweise auch eine Phasengrenze im physikalischen Sinn darstellt.

Die Erfinder haben festgestellt, daß die Zusammensetzungen gemäß der vorliegenden Erfindung auch nach mehrmaligem Schütteln, d.h. mehrmaliger Überführung in eine Emulsion, und anschließender Auftrennung der Schichten, eine deutliche Trennung der Schichten und somit eine scharfe Grenzfläche zwischen den Schichten aufweisen. Unter einer scharfen Grenzfläche ist insbesondere zu verstehen, daß ein durchschnittlicher Betrachter aus einer Distanz von etwa 0,5 Metern eine optisch scharfe Abgrenzung zwischen den Schichten wahrnimmt.

In einer bevorzugten Ausführungsform der Erfindung ist die Zusammensetzung daher dadurch gekennzeichnet, daß eine obere Emulsionsschicht und eine untere wäßrige Schicht durch eine scharfe Grenzfläche gegeneinander abgegrenzt sind.

Die erfindungsgemäßen Zusammensetzungen zeichnen sich weiterhin dadurch aus, daß auch nach mehrmaligem Schütteln, d.h. mehrmaliger Überführung in eine Emulsion, und anschließender Auftrennung der Schichten, die untere wäßrige Schicht allenfalls geringfügige Trübungen aufweist, vorzugsweise aber visuell klar ist. Unter visuell klar ist insbesondere zu verstehen, daß die Schicht wasserklar oder aber transluzent ist, d.h. gegebenenfalls einen Tyndall-Effekt aufweisen kann.

Der Vorgang der Wiederauftrennung der Schichten einer erfindungsgemäßen Zusammensetzung nach dem Schütteln ist näherungsweise vergleichbar mit dem "Aufrahmen" einer herkömmlichen Emulsion. Während jedoch beim Aufrahmen einer herkömmlichen Emulsion durch fortschreitende Koaleszenz der emulgierten Tröpfchen schließlich zwei durch eine Phasengrenze getrennte kontinuierliche Phasen entstehen, kommt es bei den erfindungsgemäßen Zusammensetzungen praktisch zu einem "Einfrieren" eines Zwischenzustands der aufrahmenden Emulsion mit der Bildung eines stabilen Systems aus unterer wäßriger Schicht und oberer Emulsionsschicht. Die erfindungsgemäßen mindestens zweischichtigen Zusammensetzungen sind lagerstabil, d.h. im Ruhezustand zeitlich stabil. Die unterschiedlichen Phasen sind durch mindestens eine Phasengrenze getrennt, deren Lage sich über einen längeren Betrachtungszeitraum nicht wesentlich verändert.

Nach der Lehre der Erfindung lassen sich Zusammensetzungen herstellen, in welchen das Volumenverhältnis zwischen oberer Emulsionsschicht und unterer wäßriger Schicht in einem weiten Umfang variiert werden kann. Beispielsweise kann das Volumenverhältnis zwischen oberer Emulsionsschicht und unterer wäßriger Schicht von 90 : 10 bis 10 : 90, vorzugsweise 75 : 25 bis 25 : 75, und insbesondere 65 : 35 bis 35 : 65 betragen.

Ein besonderer Vorteil der erfindungsgemäßen Zusammensetzungen liegt darin, daß sich die nach dem Schütteln der Zusammensetzung gebildete Emulsion im Ruhezustand innerhalb eines kurzen Zeitraums unter Rückbildung des Ausgangszustands der Zusammensetzung entmischt. Im allgemeinen ist bereits nach etwa 15 Minuten Ruhezeit eine deutlich wahrnehmbare Trennung der Schichten zu erkennen.

In einer bevorzugten Ausführungsformen der Erfindung sind die beiden Schichten innerhalb eines Zeitraums von 5 bis 90 Minuten, vorzugsweise jedoch von 5 bis 30 Minuten zu mindestens 60%, und besonders bevorzugt zu mindestens 80% getrennt. Unter einer 80%igen Trennung ist dabei zu verstehen, daß die Vertikalausdehnung der unteren wäßrigen Schicht 80% der Vertikalausdehnung der unteren wäßrigen Schicht im stationären Ruhezustand beträgt. Der stationäre Ruhezustand der Zusammensetzung ist dann erreicht, wenn sich die Vertikalausdehnungen der unteren und der oberen Phase nicht weiter verändern, d.h. nach Ende des Trennens der Schichten. Dieser Ruhezustand wird in bevorzugten Ausführungsformen der Erfindung nach etwa 15 Minuten bis etwa 2 Stunden, insbesondere aber nach etwa 30 bis 60 Minuten erreicht.

Ein weiterer sehr wesentlicher Vorteil der erfindungsgemäßen Zusammensetzungen liegt darin, daß die Emulsionströpfchen der oberen Schicht sehr stabil gegen eine Koagulation sind. Selbst nach mehrfacher Wiederholung von Schütteln und Entmischen bzw. nach längerem Lagern ist keine negative Veränderung des Erscheinungsbildes der zweischichtigen Zusammensetzungen festzustellen, und insbesondere kommt es zu keiner Bildung von "Fettaugen" oder sogar öliger Schichten an der Oberfläche der Zusammensetzungen.

Die erfindungsgemäßen Zusammensetzungen enthalten als zwingende Komponente mindestens ein wasserlösliches Polymer. Unter wasserlöslich ist hier eine Löslichkeit bei 20°C von mindestens 0,5 Gew.-% zu verstehen, bezogen auf das Gesamtgewicht der Lösung des Polymers in Wasser.

Als wasserlösliche Polymere eignen sich alle in Wasser bei 20° C in Mengen von wenigstens 0,5 Gew.-% löslichen natürlichen oder synthetischen hochmolekularen Stoffe mit wiederkehrenden Strukturelementen. Als natürliche Polymere werden dabei wasserlösliche Polysaccharide oder die wasserlöslichen Derivate von wasserunlöslichen Polysacchariden verstanden. Geeignete natürliche Polymere sind z.B. Stärke, Guar, Celluloseether, z.B. Methylcellulosen, Hydroxyethylcellulosen oder Carboxymethylcellulosen.

Geeignete synthetische Polymerverbindungen sind z.B. Polyvinylalkohole, Polyvinylpyrrolidon, Polyacrylamide, Polyvinylacetate und Polyalkylenglykole mit Molekulargewichten von mehr als 1000 D. Auch wasserlösliche Copolymerisate der Acrylsäure, des Acrylamids, des Vinylpyrrolidons und anderer wasserlöslicher Monomeren mit nicht-wasserlöslichen Comonomeren sind als Polymerverbindungen im Sinne der vorliegenden Erfindung geeignet.

Bevorzugt sind die wasserlöslichen Polymere ausgewählt aus Polyethylenglykolen, Polypropylenglykolen, EO-PO-Polyalkylenglykolen und Polyvinylpyrrolidon mit einem mittleren Molekulargewicht von mindestens 1000 D und besonders bevorzugt von 5000 bis 50000 D, wobei sich die genannten Werte auf mittels der Gelpermeation bestimmte Molekulargewichtswerte beziehen.

Als wasserlösliche Polymere besonders bevorzugt sind Polyethylenglykol und Polyvinylpyrrolidon mit einem mittleren Molekulargewicht im Bereich von 5000 bis 50000 D.

Die zur Herstellung der erfindungsgemäßen Zusammensetzungen erforderlichen Mengen an Polymerverbindungen sind von der Art und dem Molekulargewicht der Polymerverbindung abhängig, dabei gilt allgemein, daß die erforderlichen Mengen um so niedriger sind, je höher das Molekulargewicht der Polymerverbindung ist. Die Obergrenze des Gehalts an Polymerverbindungen ist so zu wählen, daß die Zubereitung nicht geliert und die Entmischung der nach Schütteln gebildeten Emulsion durch die Zähigkeit der Phasen nicht behindert wird.

Eine erfindungsgemäße Zusammensetzung enthält das wasserlösliche Polymer in einer Menge von 0,5 bis 20 Gew.-%, vorzugsweise von 1 bis 10 Gew.-%, bezogen auf das Gesamtgewicht der Zusammensetzung. Enthält die Zusammensetzung ein Gemisch aus mehreren wasserlöslichen Polymeren, so beziehen sich diese Mengenangaben jeweils auf die Gesamtheit der wasserlöslichen Polymeren.

Die erfindungsgemäßen Zusammensetzungen enthalten als weitere zwingende Komponente mindestens einen Emulgator, insbesondere einen Emulgator mit einem HLB-Wert von 8 bis 18 und vorzugsweise von 10 bis 18.

Als Emulgatoren eignen sich beispielsweise
- Anlagerungsprodukte von 4 bis 150 Mol Ethylenoxid und/oder 0 bis 5 Mol Propylenoxid an lineare Fettalkohole mit 8 bis 22 C-Atomen oder an Fettsäuren mit 12 bis 22 C-Atomen in der Alkylgruppe
- C₁₂-C₂₂-Fettsäuremono- und -diester von Anlagerungsprodukten von 1 bis 150 Mol Ethylenoxid an Polyole mit 3 bis 6 Kohlenstoffatomen, insbesondere an Glycerin,
- C₈-C₂₂-Alkylmono- und -oligoglycoside und deren ethoxylierte Analoga.
- Anlagerungsprodukte von 5 bis 150 Mol Ethylenoxid an Rizinusöl oder gehärtetes Rizinusöl,
- Partialester von Polyolen mit 3-6 Kohlenstoffatomen mit gesättigten Fettsäuren mit 8 bis 22 C-Atomen,
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen wie Sorbit, die ggf. mit 4 bis 150 Mol Ethylenoxid und/oder 0 bis 5 Mol Propylenoxid alkoxyliert sein können
- Lineare und verzweigte Fettsäuren mit 8 bis 30 C-Atomen und deren Na-, K-, Ammonium-, Ca-, Mg- und Zn-Salze.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 bis 18 können erfindungsgemäß besonders bevorzugt sein.

Als Emulgatoren besonders bevorzugt sind:
- Anlagerungsprodukte von 4 bis 150 Mol Ethylenoxid und/oder 0 bis 5 Mol Propylenoxid an lineare Fettalkohole mit 8 bis 22 C-Atomen oder an Fettsäuren mit 12 bis 22 C-Atomen in der Alkylgruppe
- Anlagerungsprodukte von 5 bis 150 Mol Ethylenoxid an Rizinusöl oder gehärtetes Rizinusöl.
- Fettsäureester von Zuckern und Zuckeralkoholen wie Sorbit, die ggf. mit 4 bis 150 Mol Ethylenoxid und/oder 0 bis 5 Mol Propylenoxid alkoxyliert sein können.

Eine erfindungsgemäße Zusammensetzung enthält den Emulgator in einer Menge von 0,02 bis 5 Gew.-%, vorzugsweise von 0,1 bis 2 Gew.-%, bezogen auf das Gesamtgewicht der Zusammensetzung. Enthält die Zusammensetzung ein Gemisch aus mehreren Emulgatoren, so beziehen sich diese Mengenangaben jeweils auf die Gesamtheit der Emulgatoren.

Erfindungsgemäß weiterhin bevorzugt ist es, wenn in der Zusammensetzung das Gewichtsverhältnis zwischen wasserlöslichem Polymer und Emulgator 1 bis 30, vorzugsweise 1 bis 10 beträgt. Sofern mehrere wasserlösliche Polymere und/oder mehrere Emulgatoren in der Zusammensetzung enthalten sind, gelten diese Werte jeweils für das Verhältnis zwischen der Gesamtheit der wasserlöslichen Polymeren und Emulgatoren.

Die erfindungsgemäßen Zusammensetzungen enthalten als weitere zwingende Komponente mindestens ein Öl, beispielsweise ein natürliches oder synthetisches kosmetisches Öl aus der Gruppe der:
- pflanzlichen Öle. Beispiele für solche Öle sind Sonnenblumenöl, Olivenöl, Sojaöl, Rapsöl, Mandelöl, Jojobaöl, Weizenkeimöl, und die flüssigen Anteile des Kokosöls. Geeignet sind aber auch andere Triglyceridöle wie die flüssigen Anteile des Rindertalgs sowie synthetische Triglyceridöle.
- flüssigen Paraffinöle, Isoparaffinöle und synthetischen Kohlenwasserstoffe sowie Di-n-alkylethern mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-isopentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Din-octylether (Cetiol^{®} OE) können bevorzugt sein.
- Esteröle. Unter Esterölen sind zu verstehen die Ester von C₆ - C₃₀ - Fettsäuren mit C₂ - C₃₀- Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, lsotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), (s.u.) Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Diisopropyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrischen, unsymmetrischen oder cyclischen Ester der Kohlensäure mit Fettalkoholen, beispielsweise beschrieben in der DE-OS 197 56 454, Glycerincarbonat oder Dicaprylylcarbonat (Cetiol^{®} CC).

Als Öle besonders bevorzugt sind Hexyllaurat, Di-n-butyladipat, Di-n-octylether, Isopropylmyristat, Isopropylpalmitat und 1,3-Di-(2-ethyl-hexyl)-cyclohexan.

Eine erfindungsgemäße Zusammensetzung enthält das Öl in einer Menge von 5 bis 40 Gew.-%, vorzugsweise von 10 bis 25 Gew.-%, bezogen auf das Gesamtgewicht der Zusammensetzung. Enthält die Zusammensetzung ein Gemisch aus mehreren Ölen, so beziehen sich diese Mengenangaben jeweils auf die Gesamtheit der Öle.

Als weitere Komponente kann in der erfindungsgemäßen Zusammensetzung ein Alkohol oder ein Gemisch mehrerer Alkohole enthalten sein. Geeignet sind vorzugsweise Alkohole aus der Gruppe der ein- oder mehrwertigen Alkohole mit 2 bis 10 Kohlenstoffatomen, wie z.B. Ethanol, 1-Propanol, 2-Propanol, n-Butanol, Isobutanol, Methoxyethanol oder 2-Ethoxyethanol. Geeignete Diole sind z.B. Ethylenglycol, 1,2-Propylenglycol, 1,4-Butandiol, 1,5-Pentandiol, 1,6-Hexandiol, Diethylenglycol, Triethylenglycol, Dipropylenglycol, Glycerinmonomethylether oder Cyclohexandiol. Geeignete Polyole sind Glycerin, Erythrit, Diglycerin, Triglycerin, Sorbit, Methylglucosid, Butylglucosid, Pentaerythrit oder Trimethylpropan.

Als Alkohole besonders bevorzugt sind Ethanol, 1-Propanol, 2-Propanol, 1,2-Propylenglycol und Glycerin.

Eine erfindungsgemäße Zusammensetzung kann den Alkohol in einer Menge von bis zu 40 Gew.-%, vorzugsweise bis zu 25 Gew.-% enthalten, bezogen auf das Gesamtgewicht der Zusammensetzung. Die Menge an Alkohol ist so zu beschränken, dass sich die Ölphase nicht soweit in der alkoholisch-wäßrigen Phase löst, daß keine ausreichende Menge an Ölphase für die obere Emulsionsschicht der erfindungsgemäßen Zusammensetzung verbleibt.

Enthält die Zusammensetzung ein Gemisch aus mehreren Alkoholen, so beziehen sich diese Mengenangaben jeweils auf die Gesamtheit der Alkohole.

Als weitere Komponente ist in der erfindungsgemäßen Zusammensetzung bis zu 5 Gew.-%, bezogen auf das Gesamtgewicht der Zusammensetzung, eines Salzes oder eines Gemisches mehrerer Salze aus der Gruppe der Alkalimetall-, Erdalkalimetall- und Ammoniumsalze der Schwefelsäure, der Halogenwasserstoffsäuren sowie der Genußsäuren, sowie deren Mischungen, enthalten, insbesondere Natriumchlorid, Natriumsulfat, Magnesiumsulfat, Natriumcitrat, Kaliumcitrat und Natriumlactat.

Enthält die Zusammensetzung ein Gemisch aus mehreren Salzen, so beziehen sich diese Mengenangaben jeweils auf die Gesamtheit der Salze.

In einer bevorzugten Ausführungsform der Erfindung enthält die Zusammensetzung
a) 10 bis 25 Gew.-% mindestens eines Öls
b) 1 bis 10 Gew.-% mindestens eines wasserlöslichen Polymers
c) 0,1 bis 2 Gew.-% mindestens eines Emulgators
d) 50 bis 88,9 Gew.-% Wasser,
wobei sich die Prozentangaben jeweils auf das Gesamtgewicht der Zusammensetzung beziehen.

In einer weiteren bevorzugten Ausführungsform der Erfindung enthält die Zusammensetzung
a) 10 bis 25 Gew.-% mindestens eines Öls
b) 1 bis 10 Gew.-% mindestens eines wasserlöslichen Polymers
c) 0,1 bis 2 Gew.-% mindestens eines Emulgators
d) 0,1 bis 5 Gew.-% mindestens eines Salzes
e) 50 bis 88,8 Gew.-% Wasser,
wobei sich die Prozentangaben jeweils auf das Gesamtgewicht der Zusammensetzung beziehen.

In einer weiteren bevorzugten Ausführungsform der Erfindung enthält die Zusammensetzung
a) 10 bis 25 Gew.-% mindestens eines Öls
b) 1 bis 10 Gew.-% mindestens eines wasserlöslichen Polymers
c) 0,1 bis 2 Gew.-% mindestens eines Emulgators
d) 10 bis 25 Gew.-% mindestens eines Alkohols
e) 30 bis 78,9 Gew.-% Wasser,
wobei sich die Prozentangaben jeweils auf das Gesamtgewicht der Zusammensetzung beziehen.

In einer weiteren bevorzugten Ausführungsform der Erfindung enthält die Zusammensetzung
a) 10 bis 25 Gew.-% mindestens eines Öls
b) 1 bis 10 Gew.-% mindestens eines wasserlöslichen Polymers
c) 0,1 bis 2 Gew.-% mindestens eines Emulgators
d) 10 bis 25 Gew.-% mindestens eines Alkohols
e) 0,1 bis 5 Gew.-% mindestens eines Salzes
f) 25 bis 78,8 Gew.-% Wasser,
wobei sich die Prozentangaben jeweils auf das Gesamtgewicht der Zusammensetzung beziehen.

In einer besonders bevorzugten Ausführungsform der Erfindung enthält die Zusammensetzung
a) 15 bis 25 Gew.-% mindestens eines Öls
b) 4 bis 10 Gew.-% mindestens eines wasserlöslichen Polymers
c) 0,1 bis 1 Gew.-% mindestens eines Emulgators
d) 50 bis 80,9 Gew.-% Wasser,
wobei sich die Prozentangaben jeweils auf das Gesamtgewicht der Zusammensetzung beziehen.

In einer weiteren besonders bevorzugten Ausführungsform der Erfindung enthält die Zusammensetzung
a) 15 bis 25 Gew.-% mindestens eines Öls
b) 2 bis 8 Gew.-% mindestens eines wasserlöslichen Polymers
c) 0,1 bis 1 Gew.-% mindestens eines Emulgators
d) 1 bis 5 Gew.-% mindestens eines Salzes
e) 50 bis 81,9 Gew.-% Wasser,
wobei sich die Prozentangaben jeweils auf das Gesamtgewicht der Zusammensetzung beziehen.

In einer weiteren besonders bevorzugten Ausführungsform der Erfindung enthält die Zusammensetzung
a) 15 bis 25 Gew.-% mindestens eines Öls
b) 4 bis 10 Gew.-% mindestens eines wasserlöslichen Polymers
c) 0,1 bis 1 Gew.-% mindestens eines Emulgators
d) 10 bis 20 Gew.-% mindestens eines Alkohols
e) 35 bis 70,9 Gew.-% Wasser,
wobei sich die Prozentangaben jeweils auf das Gesamtgewicht der Zusammensetzung beziehen.

In einer weiteren besonders bevorzugten Ausführungsform der Erfindung enthält die Zusammensetzung
a) 15 bis 25 Gew.-% mindestens eines Öls
b) 1 bis 6 Gew.-% mindestens eines wasserlöslichen Polymers
c) 0,1 bis 1 Gew.-% mindestens eines Emulgators
d) 10 bis 20 Gew.-% mindestens eines Alkohols
e) 1 bis 5 Gew.-% mindestens eines Salzes
f) 30 bis 72,9 Gew.-% Wasser,
wobei sich die Prozentangaben jeweils auf das Gesamtgewicht der Zusammensetzung beziehen.

In einer weiteren besonders bevorzugten Ausführungsform der Erfindung enthält die Zusammensetzung
a) 10 bis 25 Gew.-% mindestens eines Öls aus der Gruppe der Esteröle aus einer C₈-C₁₆-Fettsäure mit einem C₂-C₈-Fettalkohol.
b) 1 bis 10 Gew.-% mindestens eines wasserlöslichen Polymers aus der Gruppe der Polyethylenglykole mit einem Molekulargewicht von 5000-25000 Dalton
c) 0,1 bis 2 Gew.-% mindestens eines Emulgators aus der Gruppe der gehärteten Rizinusöle mit mehr als 25 EO-Einheiten
d) 40 bis 88,9 Gew.-% Wasser,
wobei sich die Prozentangaben jeweils auf das Gesamtgewicht der Zusammensetzung beziehen.

In einer weiteren besonders bevorzugten Ausführungsform der Erfindung enthält die Zusammensetzung
a) 10 bis 25 Gew.-% mindestens eines Öls aus der Gruppe der Esteröle aus einer C₈-C₆-Fettsäure mit einem C₂-C₈-Fettalkohol.
b) 1 bis 10 Gew.-% mindestens eines wasserlöslichen Polymers aus der Gruppe der Polyethylenglykole mit einem Molekulargewicht von 5000-25000 Dalton
c) 0,1 bis 2 Gew.-% mindestens eines Emulgators aus der Gruppe der gehärteten Rizinusöle mit mehr als 25 EO-Einheiten
d) 10 bis 25 Gew.-% Ethanol
e) 0,1 bis 5 Gew.-% mindestens eines Salzes aus der Gruppe der Alkali- und Erdalkalisulfate
f) 25 bis 78,8 Gew.-% Wasser,
wobei sich die Prozentangaben jeweils auf das Gesamtgewicht der Zusammensetzung beziehen.

Neben den genannten Komponenten können die erfindungsgemäßen Zusammensetzungen je nach ihrem Einsatzzweck auch weitere, für die Anwendung oder das Aussehen vorteilhafte Komponenten enthalten, z.B. solche, wie sie in kosmetischen Reinigungs- und Pflegemitteln üblich sind. Dies sind z.B. Farbstoffe, Duftstoffe, Konservierungsmittel und pH-Stellmittel (Puffersalze), Komplexbildner, Antioxidantien und kosmetische oder dermatologische Wirksubstanzen.

Von besonderer Bedeutung ist der Zusatz von einem oder mehreren Farbstoffen, bevorzugt von solchen, die in den entmischten Schichten eine unterschiedliche Löslichkeit aufweisen und auf diese Weise den Zusammensetzungen ein besonders ansprechendes Aussehen verleihen.

In einer weiteren bevorzugten Ausführungsform der Erfindung enthalten die Zusammensetzungen ein Parfüm in einer Menge von 0,1 bis 5, vorzugsweise 0,5 bis 2 Gew.-%, bezogen auf das Gesamtgewicht der Zusammensetzung. Das Parfüm liegt überwiegend in der oberen Emulsionsschicht vor. Das bewirkt den vorteilhaften Effekt eines intensivierten Dufterlebnisses beim Riechen an einem oben offenen bzw. geöffneten, eine solche erfindungsgemäße Zusammensetzung enthaltenden Behältnis.

Die erfindungsgemäßen Zusammensetzungen können durch Aufmischen unmittelbar aus ihren Rohstoffen, anschließendes Durchmischen und abschließendes Stehen des Mittels zur Auftrennung der temporären Emulsion hergestellt werden.

Die erfindungsgemäßen Zusammensetzungen können z.B. als Reinigungs- oder Pflegemittel, insbesondere als kosmetische Reinigungs- oder Pflegemittel verwendet werden. Besonders bevorzugt ist ihre Verwendung als Mittel zur kosmetischen Pflege und/oder Behandlung der Haut.

Die erfindungsgemäßen Zusammensetzungen sind sprühbar und können daher in einem Sprühspender eingesetzt werden.

Ein weiterer Gegenstand der Erfindung ist demgemäß ein Erzeugnis, enthaltend eine erfindungsgemäße Zusammensetzung und einen Sprühspender.

Die folgenden Beispiele sollen den Gegenstand der Erfindung näher erläutern, ohne ihn darauf zu beschränken:
**Rezepturen** (Alle Prozentangaben in Gew.-%):

### Beispiel 1:

a) Di-n-Butyladipat 20,0 %
b) Polyvinylpyrrolidon, Molgewicht = 17000 D 8,0 %
c) Polyoxyethylen-20- Cetylstearylalkohol 0,5 %
d) Wasser (vollentsalzt) 71,5 %

### Beispiel 2:

a) Di-n-Octylether 15,0 %
b) Polyethylenglykol, Molgewicht = 12000 D 8,0 %
c) Polyoxyethylen-20- Sorbitanmonolaurat 0,4 %
d) Kaliumcitrat 5,0 %
e) Wasser (vollentsalzt) 71,6 %

### Beispiel 3:

a) 1,3-Di-(2-ethyl-hexyl)-cyclohexan 18,0 %
b) Polyvinylpyrrolidon, Molgewicht = 17000 D 8,0 %
c) Polyoxyethylen-40- Rizinusöl (gehärtet) 0,1 %
d) Ethanol (mit 0,5% Diethylphthalat vergällt) 15,0 %
e) Wasser (vollentsalzt) 58,9 %

### Beispiel 4:

a) Isopropylmyristat 10,0 %
b) Di-n-Octyl Ether 10,0 %
c) Polyethylenglykol, Molgewicht = 12000 D 4,0 %
d) Polyoxyethylen-20- Cetylstearylalkohol 0,4 %
e) Natriumchlorid 2,0 %
f) Ethanol (mit 0,5% Diethylphthalat vergällt) 15,0 %
g) Wasser (vollentsalzt) 58,6 %

### Herstellung der visuell homogenen Emulsionen:

Zunächst wurden bei 20°C aus den Rezepturbestandteilen folgende getrennte Mischungen hergestellt:
a) Ölphase: Öl bzw. Mischung der Öle
b) wäßrige Phase: Wasser, Polymer, Emulgator, ggf. Salz, ggf. Ethanol.
Beide Phasen (insgesamt 75g je Beispiel) wurden mittels Laborrührer (IKA Eurostar) und Flügelrührer (3 cm Flügelbreite) in einer Weithalsflasche (125 ml) bei 500 U/min 5 min bei 20°C emulgiert.

### Trenngeschwindigkeit der Emulsionen

Beim Ruhen der wie vorstehend hergestellten Emulsion erfolgte eine Auftrennung in eine untere wäßrige Schicht und eine obere Emulsionsschicht. In der folgenden Tabelle sind die Zeiten aufgeführt, nach welchen beide Schichten zu 80% getrennt waren. Unter einer 80%igen Trennung ist dabei zu verstehen, daß die Vertikalausdehnung der unteren wäßrigen Schicht 80% der Vertikalausdehnung der unteren wäßrigen Schicht im stationären Ruhezustand beträgt. Der stationäre Ruhezustand der Zusammensetzung ist dann erreicht, wenn sich die Vertikalausdehnungen der unteren und der oberen Phase nicht weiter verändern, d.h. nach Ende des Trennens der Schichten.

| Beispiel Nr. | Zeit |
|---|---|
| 1 | 20 min |
| 2 | 20 min |
| 3 | 10 min |
| 4 | 30 min |

Die vollständige Trennung in zwei visuell unterschiedene Schichten war nach ca. 3-4 h erreicht, wobei die untere Schicht noch leicht trübe sein konnte. Eine visuelle Transparenz der unteren wäßrigen Schicht dieser Beispiel-Emulsionen war nach ca. 24 h erreicht.

Wenn man bei der Herstellung der Emulsionen Emulgierverfahren unter Einbringung höherer Scherenergien verwendete, wie z.B. Rotor-Stator-Systeme (Ultraturrax), Ultraschall oder Hochdruckhomogenisation, so erhielt man feinteiligere Emulsionen, die eine deutlich längere Zeit zur Schichttrennung benötigen.

### Stabilität (Koagulation) der oberen Emulsionsphase

In der folgenden Tabelle sind die Umdrehungsgeschwindigkeiten einer Zentrifuge (Heraeus Varifuge 3.0 R) aufgeführt, bei der noch keine Koagulation der oberen Emulsionsschicht stattfand, d.h. wo diese noch stabil war. Für diesen Test wurden 10 ml der handgeschüttelten Emulsion in Zentrifugengläser (D=13 mm, H=120 mm) gefüllt und jeweils für 5 min bei ansteigender Geschwindigkeit bei 25°C zentrifugiert. Gestartet wurde mit 500 U/min, und nach jeweils 5 min wurde die Umdrehungsgeschwindigkeit um 300 U/min gesteigert. Zwischen jeder Steigerung der Zentrifugengeschwindigkeit wurde eine visuelle Beurteilung durchgeführt.

| Beispiel-Emulsion | U/min |
|---|---|
| 1 | 1100 |
| 2 | 1100 |
| 3 | 800 |
| 4 | 1700 |

## Patentansprüche

1. Wäßrige, flüssige, visuell mindestens zweischichtige Zusammensetzung, die mindestens eine kontinuierliche wäßrige Phase I und mindestens eine diskontinuierliche ölige Phase II aufweist, wobei eine ölige Phase II zusammen mit einem Teil einer wäßrigen Phase I eine obere Emulsionsschicht, und ein weiterer Teil der wäßrigen Phase I eine untere wäßrige Schicht der Zusammensetzung bildet, die Zusammensetzung sich durch Schütteln temporär in eine visuell homogene Emulsion überführen läßt, wobei die Zusammensetzung enthält:
5 - 40 Gew.-% mindestens eines Öls,
0,02 - 5 Gew.-% mindestens eines Emulgators,
weiterhin 0,5 - 20 Ges.-% mindestens eines wasserlöslichen Polymers und bis zu 5 Gew.-% eines Salzes oder eines Gemisches mehrerer Salze aus der Gruppe der Alkalimetall-, Erdalkalimetall- und Ammoniumsalze der Schwefelsäure, der Halogenwasse-rstoffsäuren sowie der Genußsäuren, sowie deren Mischungen,
wobei sich die Prozentangaben jeweils auf das Gesamtgewicht der Zusammensetzung beziehen.

2. Zusammensetzung nach Anspruch 1, **dadurch gekennzeichnet daß** sie eine obere Emulsionsschicht und untere wäßrige Schicht in einem Volumenverhältnis von 90 : 10 bis 10 : 90, vorzugsweise 75 : 25 bis 25 : 75, insbesondere 65 : 35 bis 35 : 65 enthält.

3. Zusammensetzung nach einem der Ansprüche 1 bis 2. **dadurch gekennzeichnet, daß** das mindestens eine wasserlösliche Polymer ausgewählt ist aus der Gruppe, die gebildet wird von Polyethylenglycolen, Polypropylenglycolen, EO-PO-Polyalkylenglykolen und Polyvinylpyrrolidon mit Molekulargewichten von mindestens 1000 D, insbesondere von 5000 bis 50000 D.

4. Zusammensetzung nach einem der Ansprüche 1 bis 3, **dadurch gekennzeichnet, daß** das mindestens eine wasserlösliche Polymer in einer Menge von 1 bis 10 Gew.-% enthalten ist.

5. Zusammensetzung nach einem der Ansprüche 1 bis 4, **dadurch gekennzeichnet, daß** der mindestens eine Emulgator einen HLB-Wert von 8 bis 18 aufweist.

6. Zusammensetzung nach einem der Ansprüche 1 bis 5, **dadurch gekennzeichnet, daß** der mindestens eine Emulgator ausgewählt ist aus der Gruppe, die gebildet wird von Anlagerungsprodukten von 4 bis 150 Mol Ethylenoxid und/oder 0 bis 5 Mol Propylenoxid an lineare Fettalkohole mit 8 bis 22 C-Atomen oder an Fettsäuren mit 12 bis 22 C-Atomen, Anlagerungsprodukten von 5 bis 150 Mol Ethylenoxid an Rizinusöl oder gehärtetes Rizinusöl, sowie Fettsäureestern von Zuckern und Zuckeralkoholen wie Sorbit, welche ggf. mit 4 bis 150 Mol Ethylenoxid und/oder 0 bis 5 Mol Propylenoxid alkoxyliert sein können.

7. Zusammensetzung nach einem der Ansprüche 1 bis 6, **dadurch gekennzeichnet, daß** der mindestens eine Emulgator in einer Menge von 0,1 bis 2 Gew.-% enthalten ist.

8. Zusammensetzung nach einem der Ansprüche 1 bis 7, **dadurch gekennzeichnet, daß** das Gewichtsverhältnis zwischen wasserlöslichen(m) Polymer(en) und Emulgator(en) 1 bis 30, vorzugsweise 1 bis 10, beträgt.

9. Zusammensetzung nach einem der Ansprüche 1 bis 8, **dadurch gekennzeichnet, daß** das mindestens eine Öl ausgewählt ist aus der Gruppe, die gebildet wird von Hexyllaurat, Di-n-butyladipat, Di-n-octylether, Isopropylmyristat, Isopropylpalmitat und 1,3-Di-(2-ethyl-hexyl)-cyclohexan.

10. Zusammensetzung nach einem der Ansprüche 1 bis 9, **dadurch gekennzeichnet, daß** das mindestens eine Öl in einer Menge von 10 bis 25 Gew.-% enthalten ist.

11. Zusammensetzung nach einem der Ansprüche 1 bis 10, **dadurch gekennzeichnet, daß** sie zusätzlich einen Alkohol aus der Gruppe der ein- oder mehrwertigen Alkohole mit 1 bis 6 Kohlenstoffatomen enthält, welcher vorzugsweise in einer Menge von bis zu 25 Ges.-% enthalten ist.

12. Zusammensetzung nach einem der Ansprüche 1 bis 11, **dadurch gekennzeichnet, daß** sie zusätzlich ein Parfüm in einer Menge von 0,1 bis 5, vorzugsweise 0,5 bis 2 Gew.-% enthält.

13. Verfahren zur Herstellung einer Zusammensetzung nach einem der vorstehenden Ansprüche durch Aufmischen unmittelbar aus ihren Rohstoffen, anschließendes Durchmischen und abschließendes Stehen des Mittels zur Auftrennung der temporären Emulsion.

14. Verwendung einer wäßrigen, flüssigen, visuell mindestens zweischichtigen Zusammensetzung, die mindestens eine kontinuierliche wäßrige Phase I und mindestens eine diskontinuierliche ölige Phase II aufweist, wobei eine ölige Phase II zusammen mit einem Teil einer wäßrigen Phase I eine obere Emulsionsschicht, und ein weiterer Teil der wäßrigen Phase I eine untere wäßrige Schicht der Zusammensetzung bildet die Zusammensetzung sich durch Schütteln temporär in eine visuell homogene Emulsion überführen läßt, wobei die Zusammensetzung enthält:
5 - 40 Gew.-% mindestens eines Öls,
0,02 - 5 Gew.-% mindestens eines Emulgators,
weiterhin 0,5 - 20 Ges.-% mindestens eines wasserlöslichen Polymers und bis zu 5 Gew.-% eines Salzes oder eines Gemisches mehrerer Salze aus der Gruppe der Alkalimetall-, Erdalkalimetall- und Ammoniumsalze der Schwefelsäure, der Halogenwasserstoffsäuren sowie der Genußsäuren, sowie deren Mischungen,
wobei sich die Prozentangaben jeweils auf das Gesamtgewicht der Zusammensetzung beziehen, als kosmetisches Mittel, insbesondere zur kosmetischen Behandlung der Haut.

15. Erzeugnis, enthaltend eine Zusammensetzung nach einem der Ansprüche 1 bis 12 und einen Sprühspender.

## Claims

1. Aqueous, liquid, visually at least double-layer composition which has at least one continuous aqueous phase I and at least one discontinuous oily phase II, where an oily phase II, together with one part of an aqueous phase I, forms an upper emulsion layer, and a further part of the aqueous phase I forms a lower aqueous layer of the composition, the composition can be converted temporarily into a visually homogeneous emulsion by shaking, where the composition comprises:
5 - 40% by weight of at least one oil,
0.02 - 5% by weight of at least one emulsifier,
also 0.5 to 20% by weight of at least one watersoluble polymer and up to 5% by weight of a salt or of a mixture of two or more salts from the group of alkali metal, alkaline earth metal and ammonium salts of sulphuric acid, of hydrohalic acids and of food acids, and mixtures thereof,
where the percentages refer in each case to the total weight of the composition.

2. Composition according to Claim 1, **characterized in that** it comprises an upper emulsion layer and a lower aqueous layer in a volume ratio of from 90:10 to 10:90, preferably 75:25 to 25:75, in particular 65:35 to 35:65.

3. Composition according to one of Claims 1 to 2, **characterized in that** the at least one watersoluble polymer is selected from the group which is formed by polyethylene glycols, polypropylene glycols, EO-PO-polyalkylene glycols and polyvinylpyrrolidone with molecular weights of at least 1000 D, in particular of from 5000 to 50 000 D.

4. Composition according to one of Claims 1 to 3, **characterized in that** the at least one watersoluble polymer is present in an amount of from 1 to 10% by weight.

5. Composition according to one of Claims 1 to 4, **characterized in that** the at least one emulsifier has an HLB value of from 8 to 18.

6. Composition according to one of Claims 1 to 5, **characterized in that** the at least one emulsifier is selected from the group which is formed by addition products of from 4 to 150 mol of ethylene oxide and/or 0 to 5 mol of propylene oxide onto linear fatty alcohols having 8 to 22 carbon atoms or onto fatty acids having 12 to 22 carbon atoms, addition products of from 5 to 150 mol of ethylene oxide onto castor oil or hydrogenated castor oil, and fatty acid esters of sugars and sugar alcohols, such as sorbitol, which may optionally be alkoxylated with 4 to 150 mol of ethylene oxide and/or 0 to 5 mol of propylene oxide.

7. Composition according to one of Claims 1 to 6, **characterized in that** the at least one emulsifier is present in an amount of from 0.1 to 2% by weight.

8. Composition according to one of Claims 1 to 7, **characterized in that** the weight ratio between water-soluble polymer(s) and emulsifier(s) is 1 to 30, preferably 1 to 10.

9. Composition according to one of Claims 1 to 8, **characterized in that** the at least one oil is selected from the group which is formed by hexyl laurate, di-n-butyl adipate, di-n-octyl ether, isopropyl myristate, isopropyl palmitate and 1,3-di(2-ethylhexyl)cyclohexane.

10. Composition according to one of Claims 1 to 9, **characterized in that** the at least one oil is present in an amount of from 10 to 25% by weight.

11. Composition according to one of Claims 1 to 10, **characterized in that** it additionally comprises an alcohol from the group of monohydric or polyhydric alcohols having 1 to 6 carbon atoms which is preferably present in an amount of up to 25% by weight.

12. Composition according to one of Claims 1 to 11, **characterized in that** it additionally comprises a perfume in an amount of from 0.1 to 5% by weight, preferably 0.5 to 2% by weight.

13. Process for the preparation of a composition according to one of the preceding claims by blending directly from its raw materials, subsequent thorough mixing and final standing of the composition for separation of the temporary emulsion.

14. The use of an aqueous, liquid, visually at least double-layer composition which has at least one continuous aqueous phase I and at least one discontinuous oily phase II, where an oily phase II, together with one part of an aqueous phase I, forms an upper emulsion layer, and a further part of the aqueous phase I forms a lower aqueous layer of the composition, the composition can be converted temporarily into a visually homogeneous emulsion by shaking, where the composition comprises:
5 - 40% by weight of at least one oil,
0.02 - 5% by weight of at least one emulsifier,
also 0,5 to 20% by weight of at least one watersoluble polymer and up to 5% by weight of a salt or of a mixture of two or more salts from the group of alkali metal, alkaline earth metal and ammonium salts of sulphuric acid, of hydrohalic acids and of food acids, and mixtures thereof,
where the percentages refer in each case to the total weight of the composition, as cosmetic composition, in particular for the cosmetic treatment of the skin.

15. Product comprising a composition according to one of Claims 1 to 12 and a spray dispenser.

## Revendications

1. Composition aqueuse, liquide, visiblement au moins bicouche, qui présente au moins une phase aqueuse continue I et au moins une phase huileuse discontinue II, une phase huileuse II conjointement avec une partie d'une phase aqueuse I formant une couche d'émulsion supérieure et une autre partie de la phase aqueuse I formant une couche aqueuse inférieure de la composition, la composition pouvant être transformée temporairement en une émulsion visiblement homogène au moyen d'un secouage, la composition contenant :
5 à 40% en poids d'au moins une huile,
0,02 à 5% en poids d'au moins un émulsifiant,
en outre 0,5 à 20% en poids d'au moins un polymère hydrosoluble, et jusqu'à 5% en poids d'un sel ou d'un mélange de plusieurs sels issus du groupe des sels de métal alcalin, de métal alcalino-terreux et d'ammonium avec l'acide sulfurique, les acides halogénohydriques ainsi que les acides alimentaires, ainsi que leurs mélanges,
les indications de pourcentage se rapportant à chaque fois au poids total de la composition.

2. Composition selon la revendication 1, **caractérisée en ce qu'**elle contient une couche d'émulsion supérieure et une couche aqueuse inférieure, dans un rapport volumique de 90:10 à 10:90, de préférence de 75:25 à 25:75, en particulier de 65:35 à 35:65.

3. Composition selon l'une quelconque des revendications 1 ou 2, **caractérisée en ce qu'**au moins un polymère hydrosoluble est choisi dans le groupe, qui est formé par les polyéthylèneglycols, les polypropylèneglycols, les OE-OP-polyalkylèneglycols, et la polyvinylpyrrolidone avec des masses moléculaires d'au moins 1 000 D, en particulier de 5 000 à 50 000 D.

4. Composition selon l'une quelconque des revendications 1 à 3, **caractérisée en ce qu'**au moins un polymère hydrosoluble est contenu en une quantité de 1 à 10% en poids.

5. Composition selon l'une quelconque des revendications 1 à 4, **caractérisée en ce qu'**au moins un émulsifiant présente une valeur HLB (équilibre hydrophile-lipophile) de 8 à 18.

6. Composition selon l'une quelconque des revendications 1 à 5, **caractérisée en ce que** ledit au moins un émulsifiant est choisi dans le groupe qui est formé par les produits d'addition de 4 à 150 moles d'oxyde d'éthylène et/ou de 0 à 5 moles d'oxyde de propylène sur des alcools gras linéaires ayant de 8 à 22 atomes de C ou sur des acides gras ayant de 12 à 22 atomes de C, les produits d'addition de 5 à 150 moles d'oxyde d'éthylène sur l'huile de ricin ou l'huile de ricin durcie, ainsi que les esters d'acide gras de sucres et d'alcools de sucre comme le sorbitol, lesquels peuvent être éventuellement alcoxylés avec 4 à 150 moles d'oxyde d'éthylène et/ou de 0 à 5 moles d'oxyde de propylène.

7. Composition selon l'une quelconque des revendications 1 à 6, **caractérisée en ce que** ledit au moins un émulsifiant est contenu en une quantité de 0,1 à 2% en poids.

8. Composition selon l'une quelconque des revendications 1 à 7, **caractérisée en ce que** le rapport pondéral entre le ou les émulsifiants et le ou les polymères, hydrosolubles, est de 1 à 30, de préférence de 1 à 10.

9. Composition selon l'une quelconque des revendications 1 à 8, **caractérisée en ce que** ladite au moins une huile est choisie dans le groupe qui est formé par le laurate d'hexyle, l'adipate de di-n-butyle, l'éther di-n-octylique, le myristate d'isopropyle, le palmitate d'isopropyle, et le 1,3-di-(2-éthylhexyl)-cyclohexane.

10. Composition selon l'une quelconque des revendications 1 à 9, **caractérisée en ce que** ladite au moins une huile est contenue en une quantité de 10 à 25% en poids.

11. Composition selon l'une quelconque des revendications 1 à 10, **caractérisée en ce qu'**elle contient en sus un alcool issu du groupe des alcools mono- ou polyvalents ayant de 1 à 6 atomes de carbone, lequel est contenu de préférence en une quantité allant jusqu'à 25% en poids.

12. Composition selon l'une quelconque des revendications 1 à 11, **caractérisée en ce qu'**elle contient en sus un parfum en une quantité de 0,1 à 5, de préférence 0,5 à 2% en poids.

13. Procédé pour préparer une composition selon l'une quelconque des revendications précédentes, en mélangeant directement ses matières premières, puis en mélangeant intimement, puis en laissant finalement l'agent au repos afin de séparer l'émulsion temporaire.

14. Utilisation d'une composition aqueuse, liquide, visiblement au moins bicouche, qui présente au moins une phase aqueuse continue I est au moins une phase huileuse discontinue II, une phase huileuse II conjointement avec une partie d'une phase aqueuse I formant une couche d'émulsion supérieure et une autre partie de la phase aqueuse I formant une couche aqueuse inférieure de la composition, la composition pouvant être transformée temporairement en une émulsion visiblement homogène au moyen d'un secouage, la composition contenant :
5 à 40% en poids d'au moins une huile,
0,02 à 5% en poids d'au moins un émulsifiant,
en outre 0,5 à 20% en poids d'au moins un polymère hydrosoluble, et jusqu'à 5% en poids d'un sel ou d'un mélange de plusieurs sels issus du groupe des sels de métal alcalin, de métal alcalino-terreux et d'ammonium, avec l'acide sulfurique, les acides halogénohydriques ainsi que les acides alimentaires, ainsi que leurs mélanges,
les indications de pourcentage se rapportant à chaque fois au poids total de la composition, en tant qu'agents cosmétiques, en particulier pour le traitement cosmétique de la peau.

15. Produit contenant une composition selon l'une quelconque des revendications 1 à 12 et un atomiseur.
